(19) Europäisches Patentamt
European Patent Office
Office européen des brevets

(11) Publication number: **0 092 280**
**B1**

(12) # EUROPEAN PATENT SPECIFICATION

(45) Date of publication of patent specification: **19.04.89**

(51) Int. Cl.$^4$: **A 61 K 37/26,** C 07 K 1/06, C 07 K 5/02, C 12 P 21/02

(21) Application number: **83200530.0**

(22) Date of filing: **14.04.83**

(54) **Semi-synthetic preparation of human insulin.**

(30) Priority: **21.04.82 NL 8201650**

(43) Date of publication of application:
**26.10.83 Bulletin 83/43**

(45) Publication of the grant of the patent:
**19.04.89 Bulletin 89/16**

(84) Designated Contracting States:
**AT BE CH DE FR GB IT LI LU NL SE**

(56) References cited:
**EP-A-0 017 938**
**EP-A-0 045 187**
**GB-A-2 069 502**
**US-A-3 276 961**

**NATURE, vol. 280, no. 5721, 2nd August 1979, pages 412-413, Macmillan Journals Ltd. K. MORIHARA et al.: "Semi-synthesis of human insulin by trypsin-catalysed replacement of Ala-B30 by Thr in porcine insulin"**

(73) Proprietor: **AKZO N.V.**
**Velperweg 76**
**NL-6824 BM Arnhem (NL)**

(72) Inventor: **Van Dedem, Gijsbert Willem Karel**
**Bachlaan 23**
**Heesch (NL)**
Inventor: **Van Houdenhoven, François Egbert Abraham**
**Rijksweg 125B**
**Heesch (NL)**

(74) Representative: **Hermans, Franciscus G.M. et al**
**Patent Department AKZO N.V. Pharma Division**
**P.O. Box 20**
**NL-5340 BH Oss (NL)**

(56) References cited:
**CHEMICAL ABSTRACTS, vol. 95, no. 5, 1981, page 331, no. 38238h, Columbus, Ohio, USA K.MORIHARA: "Synthesis of human insulin. Semisynthesis from swine insulin with high yield**

**CHEMICAL ABSTRACTS, vol. 92, 1980, page 245, no. 142751u, Columbus, Ohio, USA K. MORIHARA et al.: "Achromobacter protease I-catalyzed conversion of porcine insulin into human insulin"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 092 280 B1**

## Description

The invention concerns the semi-synthetic preparation of human insulin starting from a novel modified human insulin and subsequent removal of the modifying groups to yield human insulin, the preparation of novel modified human insulin and the products obtained by this method.

Hypoglycaemia as a result of insulin deficiency in the blood (diabetes mellitus) can be treated by administering insulin, e.g. by means of injection or with the aid of a dosage pump. Particular use is made for this purpose of insulins located from the pancreas of animals such as pigs or cattle.

Insulin preparations of this kind have antigenic or allergenic activity in certain cases. This may partly be the result of the presence of impurities (e.g. pro-insulin, partial decomposition products therefrom, arginine-insulin, insulin-ethyl ester, monodeamino-insulin or insulin aggregates). In addition, the antigenic activity may also relate to structural differences between the animal and human insulins as a result of which the protein obtained from animals is rejected by the human body. Pig and human insulin, for example, differ in one amino acid, namely the carboxy-terminal amino acid of the so-called B-chain. This so-called B30 amino acid is alanine in pig insulin but threonine in the human. Pig insulin could therefore be converted into human insulin by replacing this B30 amino acid by threonine and a number of methods are known for doing so.

Nearly all known methods of course start by removing the B30 amino acid: in the case of pig insulin, the alanine is therefore removed. This produces des(B30)insulin, also often called des(Ala)insulin, DAI in brief. For subsequent processes, a number of alternative methods are known which lead to the formation of human insulin. These are based on the enzymatic or non-enzymatic bonding of threonine, in which the carboxyl group is protected, e.g. by an ester to des(B30) insulin. The protecting group should be removed after the threonine bonding. A commonly used protecting group is a tertiary butoxy group; quite drastic conditions are necessary for removing this, particularly an extreme pH, which also affect the remaining of the insulin molecule. As a result, partial degradation products of insulin result which are virtually inseparable from the desired product, human insulin. Hence a very impure insulin preparation is obtained. Purification also forms a major problem at an earlier stage, with the separation of the reacted and unreacted des(B30)insulin from the reaction mixture in which the threonine bonding has been achieved. These separations produce insufficiently pure material, particularly during large-scale preparation, except at the expense of very heavy losses.

The present invention, however, allows human insulin of great purity to be prepared without resorting to numerous separation and purification stages which reduce the yield.

The method according to the invention is characterized in that from a modified human insulin of the general formula I

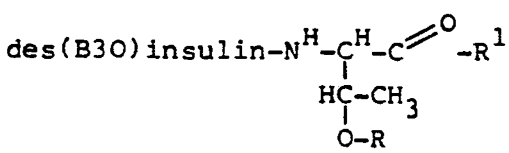

(I)

wherein

R = H or an enzymatically separable sugar residue or an ester thereof, and

$R^1$ = an enzymatically separable sugar residue or an ester thereof, or a hydrophobic or charged amino acid or di- or tripeptide comprising a hydrophobic or charged amino acid, or an ester or amide of such amino acid or peptide, or

$R^1$ = OH if R ≠ H,

the sugar and/or amino acid or peptide as defined by R and $R^1$ are enzymatically separated to yield native human insulin, after which human insulin is isolated from the medium.

The modified human insulin as described by formula I can conveniently be prepared by bonding to des(B30)insulin the compound II

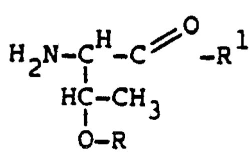

(II)

or by bonding to des(B29,B30)insulin the compound III

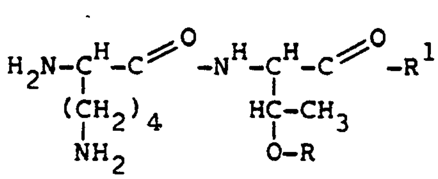

(III)

wherein R and $R^1$ in both compounds have the meaning described above.

Des(B30)insulin can be prepared separately by treating animal insulin, which is apart from the B30 amino acid on all points identical with human insulin, with a suitable enzyme, e.g. with carboxypeptidase A, or with lysylendopeptidase from Achromobacter lyticus. A des(B30)insulin residue can be prepared in situ, however, in which case the removal of the B30 amino acid and the bonding of the compound II are achieved in a so-called transpeptidase reaction by a single enzyme, e.g. trypsine.

Des(B29,B30)insulin can likewise be prepared from a suitable animal insulin using a dipeptidase, or using a peptidase which specifically cleaves the proline-lysine peptide bond.

The compound II may be bonded to the B29-lysine of the previously prepared des(B30)insulin with e.g. the aid of trypsine or with any other enzyme with trypsine-like effect, such as lysylendopeptidase. Carrier linked enzymes may be used, in which case enzyme and solution with modified insulin can be separated simply after the reaction has ended.

The covalent bond between group R and/or $R^1$ and the human insulin should be enzymatically cleavable.

This condition to a large extent governs the choice of the amino acid or acids or the sugar which form the group R and/or $R^1$.

If $R^1$ consists of amino acid or acids, the choice is for one amino acid residue, or for di- or tripeptides. These may comprise e.g. hydrophobic amino acids (e.g. phenylalanine or tryptophan) and/or charged amino acids (e.g. arginine or lysine). If hydrophobic amino acids are applied, purification is strongly promoted under hydrophobic conditions, whereas separation and purification under polar or ionizing conditions are improved on bonding e.g. charged amino acids.

Sugars which can be applied according to the invention as group R and/or $R^1$ are the mono-, di- or polysaccharides. Mono- and di-saccharides in particular such as glucose, fructose, mannose, galactose, sucrose, maltose, and lactose are to be preferred. Owing to their strongly polar character sugars bonded with insulin have a very favourable effect on separation and purification of the protein.

In order to prepare human insulin, the residual group R and/or $R^1$ must be removed enzymatically from the modified human insulin with the general formula I. The choice of residual group determines which enzyme or which combination of enzymes is suitable for this purpose.

If a group $R^1$ is built up from one, two or three amino acids, it could be advantageously removed with the aid of carboxy-peptidase. A carboxy-peptidase is an exopeptidase which can remove one amino acid one after the other from the peptide chain starting from the carboxy terminal, showing preference for certain amino acids.

Carboxy-peptidase A (CPA) splits off hydrophobic amino acids in particular with a preference for the aromatic amino acids phenylalanine, tyrosine and tryptophan. On the other hand, carboxy-peptidase B (CPB) preferably splits off basic amino acids. In combination with suitably chosen amino acids in the group $R^1$, CPA and/or CPB are very suitable proteolytic enzymes for use in the method according to the invention, but other carboxy-peptidases or dipeptylcarboxy-peptidases are suitable for the same purpose.

When the group $R^1$ consists of a sugar bonded via an ester bond, the cleavage of the sugar can be established by an esterase, and when the group R consists of a sugar bonded via an ether bond, an enzyme such as galactosidase which is specific for an O-glycosidic compound of this kind can be used for cleavage.

Also part of the invention are the modified human insulins with the general formula I.

The invention can be further illustrated by means of the following examples.

In the following examples, des(B30)insulin is used as a basis in each case, prepared from pig insulin according to the method described by E. W. Schmitt et al. (Hoppe Seyler's Z. Physiol. Chemie *359*, 799 (1978), the so-called des(ala)insulin (DAI)).

Example I—VI

Bonding of di- and tripeptides to DAI

The bonding is undertaken with various substrata (di- and tripeptides and HCl-salt of tripeptide) with various enzyme systems (trypsine, and carrier-linked trypsine and lysylendopeptidase).

These bondings are undertaken as follows:

(a) A buffer medium is composed from

— a 1:1 mixture of dimethylformamide with ethanol (= the so-called organic fraction) and

— a certain quantity of tris HCl buffer with concentration of 0.5 $mol.l^{-1}$.

(b) A weighed quantity of substratum is dissolved in this buffer medium together with a quantity of DAI.

(c) This mixture is raised to the desired pH by means of concentrated NaOH (1 $mol.l^{-1}$).

(d) A quantity of the bonding enzyme is added, after which, the bonding reaction commences and which then continues at 37°C.

(e) On completion of the reaction period, the reaction product is separated chromatographically; the yield of the bonding product is determined from the chromatogram and is expressed as a percentage of the theoretical value.

The reaction conditions and yield for examples I—VI appear in Table 1.

Table 1

| example nr. / conditions | I | II | III | IV | V | VI |
|---|---|---|---|---|---|---|
| concentration DAI (g.$l^{-1}$) | 67,1 | 66,6 | 13,6 | 25,2 | 32,2 | 32,2 |
| substrate | thr-phe | thr-phe | thr-phe-phe | thr-phe-phe-HCl | thr-phe-phe-HCl | thr-phe-phe |
| concentration (mol.$l^{-1}$) | 0,176 | 0,175 | 0,067 | 0,037 | 0,047 | 0,025 |
| enzyme(g.$l^{-1}$); trypsine: | 2,7 | | 5 | | | |
| trypsine silica: | | 808 | | | 157 | 157 |
| lysylendo-peptidase silica: | | | | 77 | | |
| medium (% organic fraction) | 60 | 60 | 67 | 60 | 60 | 60 |
| pH | 6,2 | 6,5 | 6,5 | 6,3 | 6,5 | 6,5 |
| reaction time (hours) | 1/3 | 1 | 7/12 | 48 | 3 | 3 |
| yield (% of theor.) | 53 | 28 | 54 | 38 | 27 | 16 |

Example VII

Chromatographic separation of bonding products

A comparison has been made of the chromatographic behaviour of peptide bonded DAI with that of ester bonded DAI.

For this purpose, the bonding products of DAI with thr-O-methyl, thr-O-tBu, thr-phe-O-methyl and thr-phe-phe, are prepared in the way described in example I—IV.

The bonding conditions were: DAI concentration 14 g.$l^{-1}$, 0,06 mol.$l^{-1}$ substratum, 2,5 g.$l^{-1}$ trypsine, 67% organic fraction in medium pH 6.4 and a reaction period of half an hour.

The bonding products were separated on an analytical HPLC column and the retention values (Rf) determined. The results of this separation appear in Table 2 as the difference in retention value of the bonding products from those of DAI (ΔRf).

Table 2

| Coupled to DAI | ΔRf (sec) |
|---|---|
| thr-O-methyl | 220 |
| thr-O-tBu | 1000 |
| thr-phe-O-methyl | 1400 |
| thr-phe-phe | 1700 |

Example VIII

Removal of bonded group phe-phe from DAI-thr-phe-phe

After separating unconverted DAI and bonding product DAI-thr-phe-phe on a hydrophobic column, a fraction of 23 ml was obtained which contained 0,85 $mg.ml^{-1}$ bonding product. This bonding product was freeze-dried and then dissolved in 0,2 $mol.l^{-1}$ ammoniumbicarbonate buffer pH 8,5 to a final concentration of approx. 2,8 $mg.ml^{-1}$. To this solution 2 μl carboxypeptidase was added (2.3 E). The reaction was stopped after 30 minutes by adding an equal volume of 0,5 $mol.l^{-1}$ citric acid. The material was then crystallised.

Examination showed that the bonding product had been completely converted to human insulin as demonstrated with the aid of amino acid analysis. After crystallisation, no other amino acids than phenylalanine could be demonstrated in the supernatant with the aid of amino acid analysis.

**Claims for the Contracting States: BE CH DE FR GB IT LI LU NL SE**

1. Method for the semi-synthetic preparation of human insulin characterized in that from a modified human insulin of the general formula:

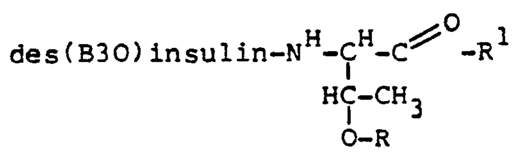

wherein

R = H or an enzymatically separable sugar residue or an ester thereof, and

$R^1$ = an enzymatically separable sugar residue or an ester thereof, or a hydrophobic or charged amino acid or a di- or tripeptide comprising a hydrophobic or charged amino acid, or an amide or ester of such amino acid or peptide, or $R^1$ = OH if R ≠ H

the sugar and/or amino acid or peptide as defined by R and/or $R^1$ are enzymatically separated, after which human insulin is isolated from the medium.

2. Method for the semi-synthetic preparation of human insulin according to claim 1 comprising bonding to des(B30)insulin or a des(B30)insulin residue the compound

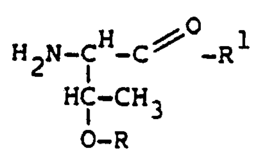

wherein R and $R^1$ have the meaning defined in claim 1, enzymatic separation of the sugar and/or amino acid or peptide as defined by R and/or $R^1$, and isolation of human insulin from the medium.

3. Method for the semi-synthetic preparation of human insulin according to claim 1 comprising bonding to des(B29,B30)insulin the compound

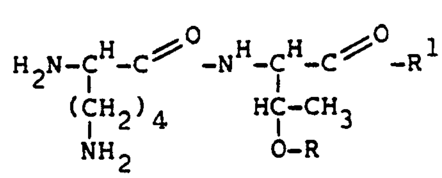

wherein R and $R^1$ have the meaning defined in claim 1, enzymatic separation of the sugar and/or amino acid or peptide as defined by R and/or $R^1$, and isolation of human insulin from the medium.

4. Method as claimed in claim 1—3, characterized in that the group R = H and $R^1$ = a hydrophobic or charged amino acid, dipeptide or tripeptide or an ester or amide thereof.

5. Method as claimed in claim 4, characterized in that the group $R^1$ = a mono-, di- or trimer of phenylalanine.

6. Method as claimed in claim 5, characterized in that the group $R^1$ is enzymatically separated using carboxypeptidase.

7. Method as claimed in claim 6, characterized in that the carboxypeptidase is carboxypeptidase A.

8. Modified human insulin characterized by the formula

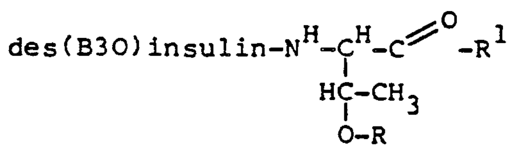

wherein R and $R^1$ have the meaning as defined in claim 1.

**Claims for the Contracting State: AT**

1. Method for the semi-synthetic preparation of human insulin characterized in that from a modified human insulin of the general formula:

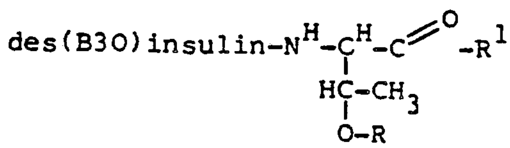

wherein

R = H or an enzymatically separable sugar residue or an ester thereof, and

$R^1$ = an enzymatically separable sugar residue or an ester thereof, or a hydrophobic or charged amino acid or a di- or tripeptide comprising a hydrophobic or charged amino acid, or an amide or ester of such amino acid or peptide, or $R^1$ = OH if $R \neq H$

the sugar and/or amino acid or peptide as defined by R and/or $R^1$ are enzymatically separated, after which human insulin is isolated from the medium.

2. Method for the semi-synthetic preparation of human insulin according to claim 1 comprising bonding to des(B30)insulin or a des(B30)insulin residue the compound

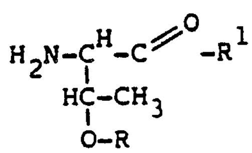

wherein R and $R^1$ have the meaning defined in claim 1, enzymatic separation of the sugar and/or amino acid or peptide as defined by R and/or $R^1$, and isolation of human insulin from the medium.

3. Method for the semi-synthetic preparation of human insulin according to claim 1 comprising bonding to des(B29,B30)insulin the compound

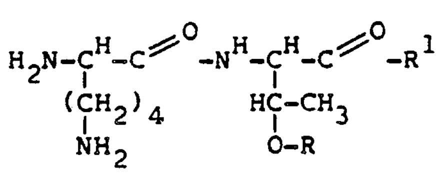

wherein R and $R^1$ have the meaning defined in claim 1, enzymatic separation of the sugar and/or amino acid or peptide as defined by R and/or $R^1$, and isolation of human insulin from the medium.

4. Method as claimed in claim 1—3, characterized in that the group R = H and $R^1$ = a hydrophobic or charged amino acid, dipeptide or tripeptide or an ester or amide thereof.

5. Method as claimed in claim 4, characterized in that the group $R^1$ = a mono-, di- or trimer of phenylalanine.

6. Method as claimed in claim 5, characterized in that the group $R^1$ is enzymatically separated using

carboxypeptidase.

7. Method as claimed in claim 6, characterized in that the carboxypeptidase is carboxypeptidase A.

**Patentansprüche für die Vertragsstaaten: BE CH DE FR GB IT LI LU NL SE**

1. Verfahren zur halbsynthetischen Herstellung von menschlichem Insulin, dadurch gekennzeichnet, dass man von einem menschlichen Insulin der allgemeinen Formel:

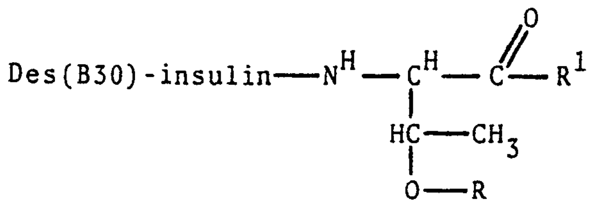

in welcher

R = H oder einen enzymatisch abtrennbaren Zuckerrest oder einen Ester davon, und

$R^1$ = einen enzymatisch abtrennbaren Zuckerrest oder einen Ester davon oder eine hydrophobe oder geladene Aminosäure oder ein Di- oder Tripeptid, welches eine hydrophobe oder geladene Aminosäure enthält, oder ein Amid oder einen Ester einer solchen Aminosäure oder Peptid oder $R^1$ = OH, falls R ≠H, darstellt,

den Zucker und/oder die Aminosäure oder das Peptid, wie durch R und/oder $R^1$ dargestellt, enzymatisch abtrennt, worauf das menschliche Insulin aus dem Medium isoliert wird.

2. Verfahren zur halbsynthetischen Herstellung von menschlichem Insulin nach Patentanspruch 1, umfassend das Binden an Des(B30)-insulin oder einen Des(B30)-insulinrest einer Verbindung

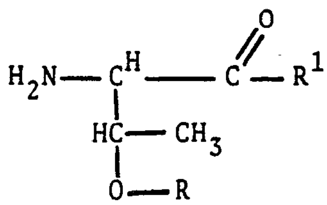

in welcher R und $R^1$ die in Patentanspruch 1 definierte Bedeutung aufweisen, enzymatische Trennung des Zuckers und/oder der Aminosäure oder des Peptides, wie durch R und/oder $R^1$ definiert, und Isolierung des menschlichen Insulins aus dem Medium.

3. Verfahren zur halbsynthetischen Herstellung von menschlichem Insulin nach Patentanspruch 1, umfassend das Binden an Des(B29),B30)-insulin der Verbindung

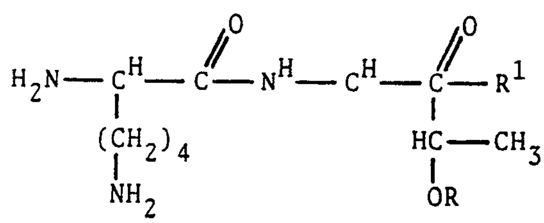

in welcher R und $R^1$ die in Patentanspruch 1 definierte Bedeutung aufweisen, enzymatische Trennung des Zuckers und/oder der Aminosäure oder des Peptides, wie durch R und/oder $R^1$ definiert, und Isolierung des menschlichen Insulins aus dem Medium.

4. Verfahren nach den Patentansprüchen 1 bis 3, dadurch gekennzeichnet, dass die Gruppe R = H und $R^1$ = eine hydrophobe oder geladene Aminosäure, ein Dipeptid oder Tripeptid oder ein Ester oder Amid davon ist.

5. Verfahren nach Patentanspruch 4, dadurch gekennzeichnet, dass die Gruppe $R^1$ = ein Mono-, Di- oder Trimer von Phenylalanin ist.

6. Verfahren nach Patentanspruch 5, dadurch gekennzeichnet, dass die Gruppe $R^1$ enzymatisch abgetrennt wird unter Verwendung von Carboxypeptidase.

7. Verfahren nach Patentanspruch 6, dadurch gekennzeichnet, dass die Carboxypeptidase Carboxypeptidase A ist.

8. Modifiziertes menschliches Insulin, gekennzeichnet durch die Formel

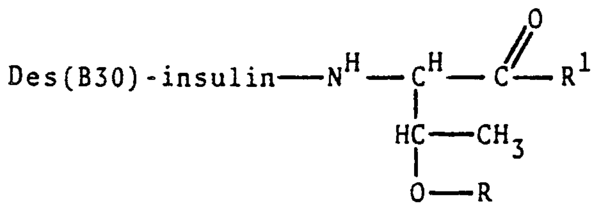

in welcher R und R¹ die in Patentanspruch 1 definierte Bedeutung aufweist.

**Patentansprüche für den Vertragsstaat: AT**

1. Verfahren zur halbsynthetischen Herstellung von menschlichem Insulin, dadurch gekennzeichnet, dass man von einem menschlichen Insulin der allgemeinen Formel:

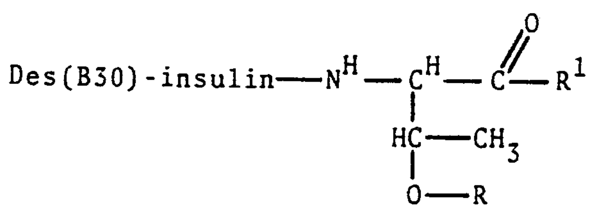

in welcher
R = H oder einen enzymatisch abtrennbaren Zuckerrest oder einen Ester davon, und
R¹ = einen enzymatisch abtrennbaren Zuckerrest oder einen Ester davon oder eine hydrophobe oder geladene Aminosäure oder ein Di- oder Tripeptid, welches eine hydrophobe oder geladene Aminosäure enthält, oder ein Amid oder einen Ester einer solchen Aminosäure oder Peptid oder R¹ = OH, falls R ≠H, darstellt,
den Zucker und/oder die Aminosäure oder das Peptid, wie durch R und/oder R¹ dargestellt, enzymatisch abtrennt, worauf das menschliche Insulin aus dem Medium isoliert wird.

2. Verfahren zur halbsynthetischen Herstellung von menschlichem Insulin nach Patentanspruch 1, umfassend das Binden an Des(B30)-insulin oder einen Des(B30)-insulinrest einer Verbindung

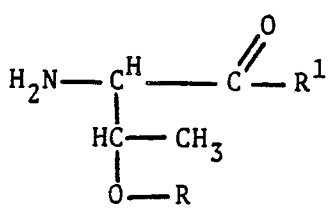

in welcher R und R¹ die in Patentanspruch 1 definierte Bedeutung aufweisen, enzymatische Trennung des Zuckers und/oder der Aminosäure oder des Peptides, wie durch R und/oder R¹ definiert, und Isolierung des menschlichen Insulins aus dem Medium.

3. Verfahren zur halbsynthetischen Herstellung von menschlichem Insulin nach Patentanspruch 1, umfassend das Binden an Des(B29),B30)-insulin der Verbindung

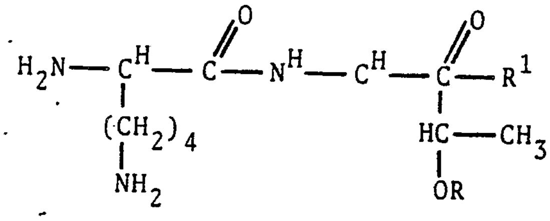

in welcher R und R¹ die in Patentanspruch 1 definierte Bedeutung aufweisen, enzymatische Trennung des

Zuckers und/oder der Aminosäure oder des Peptides, wie durch R und/oder $R^1$ definiert, und Isolierung des menschlichen Insulins aus dem Medium.

4. Verfahren nach den Patentansprüchen 1 bis 3, dadurch gekennzeichnet, dass die Gruppe R = H und $R^1$ = eine hydrophobe oder geladene Aminosäure, ein Dipeptid oder Tripeptid oder ein Ester oder Amid davon ist.

5. Verfahren nach Patentanspruch 4, dadurch gekennzeichnet, dass die Gruppe $R^1$ = ein Mono-, Di- oder Trimer von Phenylalanin ist.

6. Verfahren nach Patentanspruch 5, dadurch gekennzeichnet, dass die Gruppe $R^1$ enzymatisch abgetrennt wird unter Verwendung von Carboxypeptidase.

7. Verfahren nach Patentanspruch 6, dadurch gekennzeichnet, dass die Carboxypeptidase Carboxypeptidase A ist.

**Revendications pour les Etats contractants: BE CH DE FR GB IT LI LU NL SE**

1. Procédé pour la préparation semi-synthétique d'insuline humaine, caractérisé par le fait qu'à partir d'une insuline humaine modifiée de formule générale:

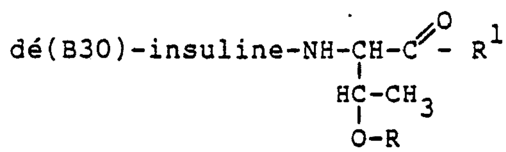

dans laquelle

R = H ou un résidu de sucre séparable par voie enzymatique ou un ester d'un tel résidu de sucre, et

$R^1$ = un résidu de sucre séparable par voie enzymatique ou un ester d'un tel résidu de sucre, ou un amino-acide hydrophobe ou chargé ou un di- ou tripeptide comprenant un amino-acide hydrophobe ou chargé, ou un amide ou ester d'un tel amino-acide ou peptide, ou bien $R^1$ = OH si $R \neq H$,

on sépare par voie enzymatique le sucre et/ou l'amino-acide ou peptide représentés par R et/ou $R^1$, après quoi on isole l'insuline humaine du milieu.

2. Procédé pour la préparation semi-synthétique d'insuline humaine selon la revendication 1, dans lequel on fixe à de la dé(B30)-insuline ou à un résidu de dé(B30)-insuline le composé

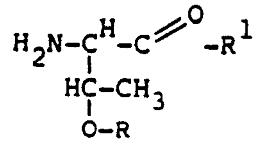

où R et $R^1$ ont les significations données dans la revendication 1, on sépare par voie enzymatique le sucre et/ou l'amino-acide ou peptide représentés par R et/ou $R^1$, et on isole l'insuline humaine du milieu.

3. Procédé pour la préparation semi-synthétique d'insuline humaine selon la revendication 1, dans lequel on fixe à de la dé(B29,B30)-insuline le composé

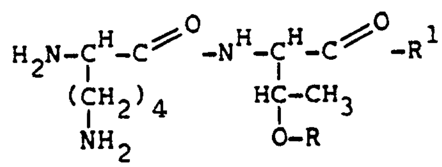

où R et $R^1$ ont les significations données dans la revendication 1, on sépare par voie enzymatique le sucre et/ou l'amino-acide ou peptide représentés par R et/ou $R^1$, et on isole l'insuline humaine du milieu.

4. Procédé tel que revendiqué dans la revendication 1, 2 ou 3, caractérisé par le fait que le groupe R = H et $R^1$ = un amino-acide hydrophobe ou chargé, un dipeptide ou tripeptide, ou un ester ou amide d'un tel amino-acide ou peptide.

5. Procédé tel que revendiqué dans la revendication 4, caractérisé par le fait que le groupe $R^1$ = un mono-, di- ou trimère de phénylalanine.

6. Procédé tel que revendiqué dans la revendication 5, caractérisé par le fait que l'on sépare le groupe $R^1$ par voie enzymatique en utilisant une carboxypeptidase.

7. Procédé tel que revendiqué dans la revendication 6, caractérisé en ce que la carboxypeptidase est une carboxypeptidase A.

8. Insuline humaine modifiée, caractérisée par la formule

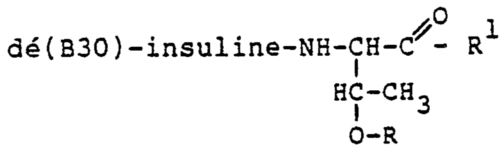

dans laquelle R et $R^1$ ont les significations données dans la revendication 1.

**Revendications pour l'Etat contractant: AT**

1. Procédé pour la préparation semi-synthétique d'insuline humaine, caractérisé par le fait qu'à partir d'une insuline humaine modifiée de formule générale:

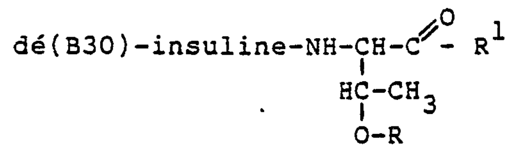

dans laquelle
R = H ou un résidu de sucre séparable par voie enzymatique ou un ester d'un tel résidu de sucre, et
$R^1$ = un résidu de sucre séparable par voie enzymatique ou un ester d'un tel résidu de sucre, ou un amino-acide hydrophobe ou chargé ou un di- ou tripeptide comprenant un amino-acide hydrophobe ou chargé, ou un amide ou ester d'un tel amino-acide ou peptide, ou bien $R^1$ = OH si $R \neq H$,
on sépare par voie enzymatique le sucre et/ou l'amino-acide ou peptide représentés par R et/ou $R^1$, après quoi on isole l'insuline humaine du milieu.

2. Procédé pour la préparation semi-synthétique d'insuline humaine selon la revendication 1, dans lequel on fixe à de la dé(B30)-insuline ou à un résidu de dé(B30)-insuline le composé

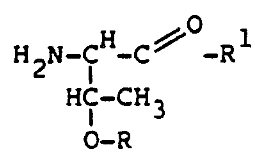

où R et $R^1$ ont les significations données dans la revendication 1, on sépare par voie enzymatique le sucre et/ou l'amino-acide ou peptide représentés par R et/ou $R^1$, et on isole l'insuline humaine du milieu.

3. Procédé pour la préparation semi-synthétique d'insuline humaine selon la revendication 1, dans lequel on fixe à de la dé(B29,B30)-insuline le composé

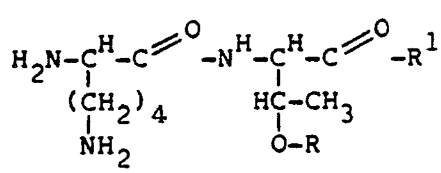

où R et $R^1$ ont les significations données dans la revendication 1, on sépare par voie enzymatique le sucre et/ou l'amino-acide ou peptide représentés par R et/ou $R^1$, et on isole l'insuline humaine du milieu.

4. Procédé tel que revendiqué dans la revendication 1, 2 ou 3, caractérisé par le fait que le groupe R = H et $R^1$ = un amino-acide hydrophobe ou chargé, un dipeptide ou tripeptide, ou un ester ou amide d'un tel amino-acide ou peptide.

5. Procédé tel que revendiqué dans la revendication 4, caractérisé par le fait que le groupe $R^1$ = un mono-, di- ou trimère de phénylalanine.

6. Procédé tel que revendiqué dans la revendication 5, caractérisé par le fait que l'on sépare le groupe $R^1$ par voie enzymatique en utilisant une carboxypeptidase.

7. Procédé tel que revendiqué dans la revendication 6, caractérisé en ce que la carboxypeptidase est une carboxypeptidase A.